(19) Europäisches Patentamt
European Patent Office
Office européen des brevets

(11) Veröffentlichungsnummer: **0 128 300 B2**

# (12) NEUE EUROPÄISCHE PATENTSCHRIFT

(45) Veröffentlichungstag der neuen Patentschrift:
27.12.91 Patentblatt 91/52

(51) Int. Cl.⁵: **A61N 5/10, A61B 6/00, H05G 1/02**

(21) Anmeldenummer: 84103996.9

(22) Anmeldetag: 10.04.84

(54) **Rohrkanalweiche, insbesondere für die medizinische Strahlentechnik.**

(30) Priorität: 16.04.83 DE 3313857

(43) Veröffentlichungstag der Anmeldung:
19.12.84 Patentblatt 84/51

(45) Bekanntmachung des Hinweises auf die Patenterteilung:
20.11.86 Patentblatt 86/47

(45) Bekanntmachung des Hinweises auf die Entscheidung über den Einspruch:
27.12.91 Patentblatt 91/52

(84) Benannte Vertragsstaaten:
FR GB IT NL

(56) Entgegenhaltungen:
CH-A- 479 311
DE-A- 1 548 185
DE-A- 2 717 341
DE-A- 2 727 359
DE-B- 1 456 761
DE-B- 1 781 388
Firmenprospekt der Firma NUCLESA über das Gerät CERVITRON II
Prospekt der Firma Atomic Energy of Canada Ltd über des Gerät Curitron CT-400

(73) Patentinhaber: ISOTOPEN-TECHNIK DR. SAUERWEIN GMBH
Bergische Strasse 16
W-5657 Haan/Rheinl. 1 (DE)

(72) Erfinder: Sauerwein, Kurt, Dr.
Kattendahl 7
W-4006 Erkrath 2 (DE)
Erfinder: Kinzer, Norbert, Dipl.-Ing.
Am Kohlenmeiler 155
W-5600 Wuppertal 22 (DE)
Erfinder: Mick, Felix W. Mick Radio-Nuclear Instruments Inc.
1470 Outlook Avenue
Bronx, N.Y. 10465 (US)

(74) Vertreter: König, Reimar, Dr.-Ing. et al
Patentanwälte Dr.-Ing. Reimar König Dipl.-Ing. Klaus Bergen Wilhelm-Tell-Strasse 14
Postfach 260162
W-4000 Düsseldorf 1 (DE)

EP 0 128 300 B2

## Beschreibung

Die Erfindung betrifft eine Weiche mit Rohrkanälen in einem Gehäuse zum wahlweisen Verbinden eines Ladekanals eines Nachladegeräts für die medizinische Strahlentechnik mit je einem von mehreren, in Applikatoren endenden Rohrkanälen, in die radioaktive Strahlenquellen eingebracht und daraus zurückgeholt werden, wobei das Gehäuse durch eine Lochscheibe abgeschlossen ist, die Lochscheibe mehrere, auf einem Halbkreis angeordnete Durchgangsbohrungen aufweist, die mit den Rohrkanälen verbunden sind, koaxial zur Achse des Halbkreises der Lochscheibe im Gehäuse eine mit einem Drehantrieb verbundene Kanalscheibe mit konzentrisch um ihre Drehachse angeordneten Ausnehmungen mit darin eingreifenden Arretierelementen und mit einer Kanalbohrung angeordnet ist, die mit dem Ladekanal fluchtend fest verbunden ist und mit einer der Durchgangsbohrung der Lochscheibe zum Fluchten gebracht werden kann.

Eine derartige Vorrichtung besitzt ein unter der Bezeichnung "Cervitron II" der Firma Nuclesa, Grand-Lancy, Schweiz, bekanntes und schematisch in der schweizerischen Patentschrift 479 311 beschriebenes Gerät.

Die radioaktiven Strahlenquellen bestehen aus Kügelchen, die mit inaktiven Kügelchen in einer bestimmten Abfolge mittels Preßluft in die Applikatoren eingebracht und daraus auch wieder zurückgeholt werden. Die Weiche ist in dem Gerät angeordnet und über doppelwandige Schläuche mit den Applikatoren über ein Zwischenlager für die radioaktiven Strahlenquellen verbunden. Die Arretierelemente bestehen aus einer axial verschiebbaren, federbelasteten Kugel, die bei jeweils fluchtender Verbindung der Bohrungen in eine der Ausnehmungen der Kanalscheibe eingreift und die Kanalscheibe positioniert. Die Kanalscheibe läßt sich mittels des Drehantriebs, der die Federbelastung der Kugel über windet, weiterdrehen.

Des weiteren ist ein Nachladegerät für die medizinische Strahlentechnik mit mehreren, in Applikatoren endenden Rohrkanälen, in die radioaktive Strahlenquellen mittels eines Kabels in den Kanälen verfahren werden, unter der Bezeichnung "Curietron CT-400" der Firma Atomic Energy of Canada Ltd., Ottawa, Canada, bekanntgeworden. Bei diesem Gerät werden die Rohrkanäle mittels einer Steckverbindung mit dem Gehäuse des Geräts verbunden. In dieser Steckverbindung ist eine Sicherheitsvorrichtung angeordnet, die ein Verfahren einer radioaktiven Strahlenquelle verhindert, wenn kein Rohrkanal an die entsprechende Steckverbindung angeschlossen ist.

Drehrohrweichen zum wahlweisen Verbinden eines ankommenden Rohres mit einem von mehreren abgehenden Rohren für pneumatische Förderleitungen sind des weiteren aus den deutschen Offenlegungsschriften 1 456 761 und 1 781 388 bekannt. In beiden Fällen stehen die Eingangsleitung und die Abgangsleitung in ständiger fester Verbindung mit der Rohrweiche; bei beiden kommt es in erster Linie auf die Dichtheit der Verbindung an, weniger auf ein genaues Fluchten.

Die bei den beiden bekannten Vorrichtungen vorhandenen Positionsfühler und Verriegelungselemente dienen ausschließlich dazu, eine rein kraftschlüssige Verriegelung herbeizuführen.

Die Geräte "Cervitron II " und "Curietron CT-400" dienen dazu, am Bestrahlungsobjekt eine dreidimensionale Strahlungsverteilung, beispielsweise bei der interstitiellen oder intracavitären Tumortherapie zu erreichen. Während sich an das "Cervitron II"- Gerät bis zu sechs Applikatoren anschließen lassen, die nacheinander mit radioaktiven Strahlenquellen beaufschlagt werden, handelt es sich bei dem "Curietron CT- 400"-Gerät um ein Mehrkanal-Nachladegerät, bei dem sich bis zu vier Applikatoren gleichzeitig mit einer radioaktiven Strahlenquelle beaufschlagen lassen.

Mehrkanalgeräte sind aus technischen Gründen auf drei bis sechs Kanäle beschränkt und entsprechend der Zahl der Kanäle sehr teuer. Sie lassen sich für Anwendungen mit mehr als sechs Applikatoren wie im Falle der Bestrahlung von Halstumoren und Brustkrebs nicht vollautomatisch bedienen, wenn die Zahl der erforderlichen Applikatoren die Zahl der Kanäle überschreitet. Bei einem Einkanalgerät die Schläuche von Applikator zu Applikator jeweils zu wechseln, ist andererseits sehr zeitaufwendig und belästigt den Patienten.

Der Erfindung liegt daher die Aufgabe zugrunde, ein einkanaliges Nachladegerät, bei dem die radioaktive Strahlenquelle mittels eines Kabels in den Kanälen verfahren wird, zu einem mehrkanaligen Nachladegerät auszubauen und dafür eine einfache und funktionstüchtige Rohrweiche mit einer automatischen Steuerung zu schaffen, die ein sehr genaues Fluchten sowie eine automatische Kontrolle der Richtigkeit und Exaktheit der jeweiligen Rohrverbindung gestattet. Weiterhin soll es auf einfache Weise möglich sein, die Benutzung einer Rohrverbindung zu verhindern, wenn ein Rohrkanal nicht oder nicht richtig angeschlossen oder an seinem freien Ende nicht oder falsch mit einem weiterführenden Rohrkanal verbunden ist.

Ausgehend von dieser Aufgabenstellung wird bei einer Weiche der eingangs erwähnten Art vorgeschlagen, daß erfindungsgemäß die radioaktiven Strahlenquellen mittels eines Kabels in den Kanälen verfahren werden, die Rohrkanäle mittels Steckverbinder direkt mit den. Durchgangsbohrungen fest und fluchtend verbindbar sind, eine steuerbare Einheit zum Drehen und Verriegeln der Kanalscheibe in Relation zu der Lochscheibe mit einem Positionsfühler und einem Elektromagneten zusammenwirkt, an dem ein Verriegelungsstift angeordnet ist, der bei jeweils fluchtenden Bohrungen in eine der Ausnehmungen eingreift, und ein federbelasteter Sicherheitsendschalter die Positionierung des gehäuseseitigen Endes des Steckverbinders kontrolliert.

Vorzugsweise besteht der Positionsfühler aus einem von der Kanalscheibe verstellbaren Potentiometer.

Mit der erfindungsgemäßen Rohrkanalweiche läßt sich der Ladekanal schnell, sicher und automatisch mit einem vorbestimmten Rohrkanal koppeln, aber auch an seinem rohrweichenseitigen Ende kontrollieren. Damit ergibt sich für Einzelkanal-Nachladegeräte, bei denen der Ladekanal eine Verbindung zu dem Strahlenschutzkörper herstellt und die Rohrkanäle als Verbindungen zu den einzelnen Applikatoren dienen, eine außerordentlich einfache, preisgünstige und sichere Möglichkeit, das verhältnismäßig preiswerte Gerät zu einem einfach zu bedienenden Mehrkanalgerät zu machen. So lassen sich auch Einzelkanalgeräte mit der erfindungsgemäßen Rohrweiche nachrüsten.

Die ortsfeste Lochscheibe, die mit der Verriegelungseinheit sowie mit einem Antriebsmotor zum Drehen der Kanalscheibe ausgestattet ist, führt zu einer besonders einfachen Handhabung der erfindungsgemäßen Rohrweiche, bei der der Ladekanal mit der drehbaren Lochscheibe verbunden ist. Die Verriegelungseinheit aus einem Elektromagneten und einem daran angeordneten Verriegelungsstift eignet sich besonders für den vorgesehenen Zweck, wenn die radial oder axial an der drehbaren Scheibe angeordneten Ausnehmungen mit einer sich nach außen konzentrisch erweiternden Zentriermündung für den beispielsweise ebenfalls für Zentrierzwecke konisch zulaufenden Arretierstift versehen sind. Die Verriegelungseinheit ist mit der Steuerung der Rohrweiche verbunde und bewirkt ein genaues Fluchten der Bohrungen.

Das von der drehbaren Kanalscheibe verstellbare Potentiometer gestattet es, die Richtigkeit der Weichenposition zu überwachen. Hierzu kann die Verstellwelle eines Drehpotentiometers von der drehbaren Kanalscheibe mittels einer konzentrischen Verbindung betätigt werden. Als Antrieb der drehbaren Kanalscheibe eignet sich vor allem ein seitlich der Kanalscheibe eingreifender Zahn- oder Riementrieb. Hierdurch ergibt sich bei der Unterbringung der Drehweiche in einem zylindrischen Gehäuse eine Exzentrizität der Drehachse, die sich in Verbindung mit einem Einkanal-Nachladegerät wählen läßt, da sie mit der exzentrischen Öffnung in der Frontplatte des Strahlerkopfes übereinstimmt. Auf diese Weise wird der Ladekanal am wenigsten belastet, und es ergibt sich eine einfache Verbindung des Strahlerkopfes mit der Rohrweiche.

Das unbeabsichtigte Benutzen eines Rohrkanals läßt sich auf einfache Weise mittels des die Durchgangsbohrungen der Lochscheibe und der Kanalscheibe kontrollierenden Sicherheitsendschalters vermeiden.

Die Erfindung wird nachfolgend anhand eines in der Zeichnung dargestellten Ausführungsbeispiels des näheren erläutert. In der Zeichnung zeigen:

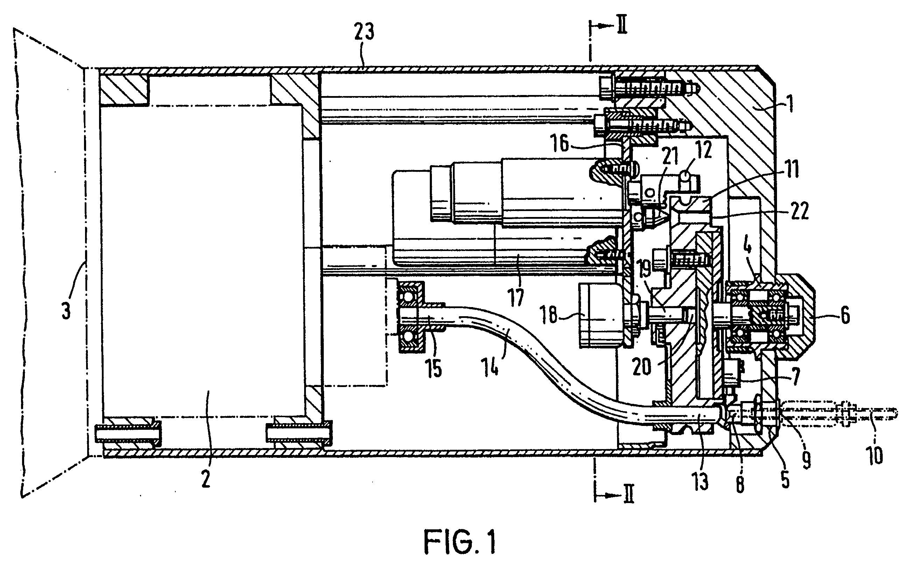

Fig. 1 eine Rohrweiche am Strahlerkopf eines Afterloading-Gerätes im axialen Teilschnitt;

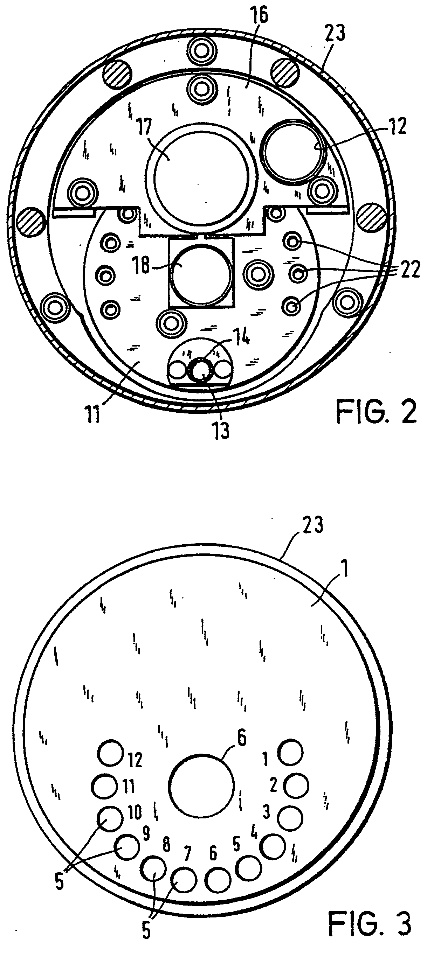

Fig. 2 eine Rohrweiche gemäss Fig. 1 im Schnitt entlang der Linie II-II sowie

Fig. 3 eine Rohrweiche gemäss Fig. 1 in Frontansicht.

Eine ortsfeste Lochscheibe 1 bildet die Stirnseite eines zylindrischen, koaxial mit einem Strahlenkopf 2 eines sonst nicht näher dargestellten Afterloading-Gerätes 3 verbundenen Gehäuses 23. Die Lochscheibe 1 weist eine aussermittige Bohrung 4 für ein Drehlager und koaxial zu dieser Bohrung zwölf etwa auf einem Halbkreis angeordnete Kanalbohrungen 5 auf. Mit Hilfe einer Schraubkappe 6 ist die Drehlagerbohrung 4 verschlossen. In die Kanalbohrungen 5 können hohle Steckverbinder 9 eingeschoben werden; sie bilden die Verbindung zu den von der Lochscheibe 1 fortführenden Rohrkanälen 10. Das vordere Ende 8 der Steckverbinder 9 betätigt bei ordnungsgemässer Steckverbindung einen Sicherheitsendschalter 7 auf der Kanalscheibe 11, der dann den Antrieb für ein in dem Kanal geführtes - in der Zeichnung nicht eigenes dargestelltes - Kabel freigibt.

Eine drehbare Kanalscheibe 11 ist in der Drehlagerbohrung 4 der ortsfesten Lochscheibe 1 frei drehbar gelagert und lässt sich mittels eines Riementriebes 12 elektromechanisch verstellen; sie weist eine mit den Kanalbohrungen 5 der ortsfesten Lochscheibe 1 zum Fluchten zu bringende Kanalbohrung 13 mit einem sich daran anschliessenden Ladekanal 14 auf, der an seinem anderen Ende mit einer konzentrisch zu der Drehlagerbohrung 4 angeordneten Mündungsöffnung 15 des Strahlerkopfes 2 fest verbunden ist.

Eine mit der ortsfesten Scheibe 1 fest verbundene Trägerplatte 16 nimmt den Motor des Riementriebes 12, einen Elektromagneten 17 und ein Drehpotentiometer 18 auf und kann mit der drehbaren Scheibe 11 exakt zum Fluchten gebracht werden. Die Drehwelle 19 des Drehpotentiometers 18 greift dann in eine zentrische Bohrung 20 der Kanalscheibe 11 ein und wird dort durch Klemmwirkung gehalten und synchron von ihr verstellt, während das Potentiometergehäuse von der Trägerplatte 16 gesichert und in üblicher Weise über elektrische Kontakte mit einer in der Zeichnung nicht dargestellten Steuereinheit verbunden ist.

Der Elektromagnet 17 weist stirnseitig einen axial verschiebbaren Arretierstift 21 auf, der nach exakter Justierung der Trägerplatte 16 in Ausnehmungen 22 eingreifen kann, die in der Kanalscheibe 11 konzentrisch zu der Drehlagerbohrung 4 angeordnet sind und zu dem Arretierstift hin eine konische Erweiterung aufweisen, deren Konuswinkel in etwa mit einer konischen Verjüngung am freien Ende des Arretierstiftes 21 korrespondiert. Die Ausnehmungen 22 korrespondieren hinsichtlich ihrer Anzahl und ihres Winkelabstandes mit den Kanalbohrungen 5 der ortsfesten Lochscheibe 1. Wenn der Arretierstift 21 in der Ausnehmung 22 steckt, muss die Kanalbohrung 13 der drehbaren Kanalscheibe 11 genau mit einer der Kanalbohrungen 5 der ortsfesten Scheibe 1 fluchten; dies wird durch ein einmaligen Justieren der Trägerplatte 16 bewirkt.

Das zylindrische Gehäuse 23 verbindet die ortsfeste Lochscheibe 1 mit dem Strahlerkopf 2; sie trägt und schützt damit alle Bauteile der Rohrweiche.

Die Rohrweiche erhält ihre Befehle von einer programmierbaren Steuereinheit, die nach einem bestimmten Zeitprogramm nicht nur dafür sorgt, dass eine in dem Afterloading-Gerät 3 strahlengeschützt angeordnete Strahlerkapsel mittels eines Kabels durch den Ladekanal 14 und mittels der Rohrweiche in einen der daran angeschlossenen Rohrkanäle 10 zu einem nicht dargestellten Applikator geführt werden kann, sondern sie steuert auch die Rohrweiche, die jeweils in der Ruheposition der Strahlerkapsel innerhalb des Afterloating-Gerätes entriegelt, in eine andere Verbindungsposition verdreht und wieder verriegelt wird. Elektrische Befehle stellen sicher, dass sich diese Schritte nur nacheinander ausführen lassen. Der Sicherheitsschalter 7 verhindert, dass der Antrieb die Strahlerkapsel aus ihrer Ruhestellung fahren kann, wenn der Ladekanal 14 mit einer der Kanalbohrungen 5 nicht fluchtet und daran kein Rohrkanal 10 angeschlossen ist.

## Patentansprüche

1. Weiche mit Rohrkanälen (10, 14) in einem Gehäuse (23) zum wahlweisen Verbinden eines Ladekanals (14) eines Nachladegeräts für die medizinische Strahlentechnik mit je einem von mehreren, in Applikatoren endenden Rohrkanälen (10), in die radioaktive Strahlenquellen eingebracht und daraus zurückgeholt werden, wobei das Gehäuse (23) durch eine Lochsheibe (1) abgeschlossen ist, die Lochscheibe (1) mehrere auf einem Halbkreis angeordnete Durchgangsbohrungen (5) aufweist, die mit den Rohrkanälen (10) verbunden sind, koaxial zur Achse des Halbkreises der Lochscheibe (1) im Gehäuse (23) eine mit einem Drehantrieb (17, 12) verbundene Kanalscheibe (11) mit konzentrisch um ihre Drehachse angeordneten Ausnehmungen (22) mit darin eingreifenden Arretierelementen (21) und mit einer Kanalbohrung (13) angeordnet ist, die mit dem Ladekanal (14) fluchtend fest verbunden ist und mit einer der Durchgangsbohrungen (5) der Lochscheibe (1) zum Fluchten gebracht werden kann, dadurch gekennzeichnet, daß die radioaktiven Strahlenquellen mittels eines Kabels in den Kanälen (10, 14) verfahren werden, die Rohrkanäle (10) mittels Steckverbinder (9) direkt mit den Durchgangsbohrungen (5) fest und fluchtend verbindbar sind, eine steuerbare Einheit (12, 17 bis 22) zum Drehen und Verriegeln der Kanalscheibe (11) in Relation zu der Lochscheibe (1) mit einem Positionsfühler (18) und einem Elektromagneten (17), an dem ein Verriegelungsstift (21) angeordnet ist, der bei jeweils fluchtenden Bohrungen (5, 13) in eine der Ausnehmungen (22) eingreift, zusammenwirkt und ein federbelasteter Sicherheitsendschalter (7) die Positionierung des gehäuseseitigen Endes des Steckverbinders (9) kontrolliert.

2. Weiche nach Anspruch 1, dadurch gekennzeichnet, daß der Positionsfühler (18) ein von der Kanalscheibe (11) verstellbares Potentiometer ist.

## Claims

1. Directing means with ducts (10, 14) in a housing (23) for selectively connecting a loading channel (14) of a reloading device for medical radiation technology to one of several ducts (10) which end in applicators and into and out of which radioactive radiation sources are introduced and retrieved, wherein the housing (23) is closed by an apertured plate (1), the apertured plate (1) comprises several through-holes (5) arranged in a semi-circle and communicating with the ducts (10), and coaxially with the axis of the semi-circle of the apertured plate (1) in the housing (23) is arranged a duct plate (11) connected to a rotary drive (17, 12), with recesses (22) arranged concentrically about its axis of rotation with locking elements (21) engaging therein and with a duct bore (13) which is rigidly connected in alignment with the loading channel (14) and can be brought into alignment with one of the through-holes (5) of the apertured plate (1), characterised in that the radioactive radiation sources are moved in the ducts (10, 14) by means of a cable, the ducts (10) can be connected rigidly and in alignment directly to the through-holes (5) by means of plug and socket connectors (9), a controllable unit (12, 17 to 22) for rotating and locking the duct plate (11) in relation to the apertured plate (1) cooperates with a position sensor (18) and an electromagnet (17) on which is mounted a locking pin (21) which engages in one of the recesses (22) whenever the bores (5, 13) are in alignment, and a spring-loaded safety limit switch (7) controls positioning of the end of the plug and socket connector (9) on the housing side.

2. Directing means according to claim 1, characterised in that the position sensor (18) is a potentiometer displaceable by the duct plate (11).

## Revendications

1. Dispositif de raccordement de conduits tubulaires (10,14) monté dans une enveloppe (23) et permettant de relier sélectivement un conduit de charge (14) d'un appareil de postchargement utilisé pour les techniques médicales relatives aux radiations à chacun des conduits d'un ensemble de conduits tubulaires (10) qui aboutissent à des applicateurs et dans lesquels des sources de rayonnement radioactif sont mises en place et retirées, l'enveloppe (13) étant fermée par un disque perforé (1) et ce disque perforé (1) comportant plusieurs alésages de passage (5) qui sont disposés sur und demi-circonférence et reliés aux conduits tubulaires (10), un disque à conduits (11), placé coaxialement à l'axe de la demi-circonférence du disque perforé (1) dans l'enveloppe (23), étant relié à un dispositif (17,12) d'entrainement en rotation et comportant des évidements (22) disposés concentriquement autour de son axe de rotation et associés à des éléments d'arrêt (21) qui s'y engagent et un alésage de conduit (13) qui est relié solidement, en respectant l'alignement, au conduit de charge (14) et qui peut être placé dans l'alignement de l'un des alésages de passage (5) du disque perforé (1), caractérisé en ce que les sources de rayonnement radioactif peuvent être déplacées dans les conduits (10,14) au moyen d'un câble, que les conduits tubulaires (10) peuvent être reliés solidement, en respectant l'alignement, directement au moyen d'une fiche de liaison (9), aux alésages de passage (5), qu'une unité (12,17 à 22) qui peut être commandée pour assurer la rotation et le verrouillage du disque à conduits (11) coopère, en relation avec le disque perforé (1), avec un palpeur de position (18) et avec un électroaimant (17) auquel est associée une goupille d'arrêt (21) qui, lorsque les alésages (5,13) sont alignés, s'engage dans l'un des évidements (22) et qu'un conjoncteur terminal de sécurité (7) soumis à l'action d'un ressort contrôle le positionnement de l'extrémité de la fiche de liaison (9) située du côté de l'enveloppe.

2. Dispositif de raccordement selon la revendication 1, caractérisé en ce que le palpeur de position (18) est un potentiomètre qui peut être déplacé par le disque à conduits (11).

FIG.1

EP 0 128 300 B2

FIG. 2

FIG. 3